# EUROPÄISCHE PATENTSCHRIFT

(11) **EP 1 659 397 B1**
(45) Veröffentlichungstag und Bekanntmachung des Hinweises auf die Patenterteilung: **03.10.2007**
(21) Anmeldenummer: 04027439.1
(22) Anmeldetag: 18.11.2004
(51) Int. Cl.: G01N 27/06

(54) **Kalibrationslösung für die Konduktometrie**
Calibration solution for conductivity measurements
Solution de calibrage pour mesures de conductivité

(43) Veröffentlichungstag der Anmeldung: 24.05.2006
(73) Patentinhaber: Hamilton Bonaduz AG, CH-7402 Bonaduz (CH)
(72) Erfinder: Bühler, Hannes, 8873 Amden (CH)
(74) Vertreter: Dey, Michael

(56) Entgegenhaltungen:
- WO-A-00/54036
- US-A- 4 092 232
- US-A- 4 996 160
- US-A- 5 472 880
- LIGHT T S ET AL: "A NEW LOW CONDUCTIVITY STANDARD SOLUTION" ANALYTICAL CHEMISTRY, AMERICAN CHEMICAL SOCIETY. COLUMBUS, US, Bd. 65, Nr. 2, 15. Januar 1993 (1993-01-15), Seiten 181-182, XP000356157 ISSN: 0003-2700

## Beschreibung

Die vorliegende Erfindung betrifft eine Lösung zur Kalibration von konduktometrischen Messzellen.

Die Messung und Kontrolle der elektrolytischen Leitfähigkeit von Lösungen ist ein wichtiger Parameter im Labor, in der Umwelt und in der Industrie. Von besonderer Bedeutung ist die Kontrolle der Leitfähigkeit im Reinstwasser, dessen Reinheit in Kraftwerken, aber auch in der Pharma- und Halbleiterindustrie von höchster Bedeutung ist. Zur Kontrolle der Messinstrumente dienen hochgenaue elektrische Widerstände, deren Verfügbarkeit und Genauigkeit keine Probleme darstellen. Zur Prüfung der Leitfähigkeitsmesszellen dienen Kalibrationslösungen mit einer genau definierten Leitfähigkeit. Solche Lösungen für den mittleren oder höheren Leitfähigkeitsbereich sind problemlos erhältlich oder können selbst hergestellt werden. Kalibrationslösungen für niedrige Leitfähigkeiten werden jedoch selten eingesetzt, da ihre Genauigkeit oftmals zu wünschen übriglässt. Weiter ist die Handhabung von solchen Lösungen schwierig und ihre Haltbarkeit ist oft sehr kurz.

Die Anforderungen an Reinstwasser sind insbesondere für Anwendungen in der Pharmaindustrie vorgeschrieben. So wird beispielsweise gemäß dem Journal "Pharmeuropa", einem Informationsmedium der Europäischen Arzneibuchkommission, für Aqua purificata eine Leitfähigkeit < 5.1 µS/cm bei 25 °C und für Aqua ad iniectabilia eine Leitfähigkeit < 1.3 µS/cm bei 25 °C gefordert.

In den U.S. Pharmacopeia (USP) 23, 24 und 25 des US-amerikanischen Arzneibuches ist eine Leitfähigkeit < 1.3 µS/cm bei 25 °C für Aqua ad iniectabilia vorgeschrieben.

Die US-"Monographs" verlangen eine Genauigkeit der Zellkonstante der Leitfähigkeitsmesszellen von ± 2 %. Diese Vorschrift ist gleichbedeutend mit der geforderten Genauigkeit der Kalibrationslösungen.

Es existieren verschiedene Normen, welche die Messmethodik und die Herstellung bzw. Handhabung der Kalibrationslösungen beschreiben.

Die Normen D 5391-93 und D 1125-95 der "American Society for Testing and Materials" (ASTM) bechreiben verschiedene Kalibrationslösungen. Die niedrigste Leitfähigkeit beträgt 146.9 µS/cm bei 25°C. Diese Lösung besteht aus einer wässrigen 0.001 M KCI Lösung.

Die Norm IEC 746, Teil 3 der "International Electrotechnical Commission" (IEC) beschreibt ebenfalls diese 0.001 M KCl-Lösung mit 146.9 µS/cm bei 25 °C.

Die Norm ISO 7888 der "International Organization for Standardization" auf internationaler Ebene bzw. die europäische Norm EN 27888 auf regionaler Ebene erwähnt ebenfalls wässrige KCI-Lösungen mit 74 und 147 µS/cm bei 25 °C. Es wird keine Stelle nach dem Komma angegeben. Um den Anforderungen dieser Normen entsprechende Lösungen herzustellen, muss das Wasser abgekocht oder durch Einleiten von Stickstoff von darin gelöstem Kohlendioxid befreit werden. Bei der Anwendung dieser Lösungen ist jeglicher Kontakt mit Luft zu unterbinden.

Entsprechende Standardlösungen mit mittleren Leitfähigkeiten im Bereich von 147 bis 12880 µS/cm werden von verschiedenen Firmen mit einer Genauigkeit zwischen 0.5 und 1 % angeboten.

Das US-metrologische Institut "National Instistute of Standards and Technology" (NIST) verkauft Kalibrationslösungen mit niedrigeren Leitwerten. Diese Lösungen mit 5, 15 und 26 µS/cm bei 25 °C sind aber sehr teuer und nicht immer lieferbar. Es existieren auch etliche Firmen, welche Standardlösungen mit geringer Leitfähigkeit von 10 und 100 µS/cm bei 25 °C anbieten. Die Industrie ist mit diesen Lösungen nicht zufrieden, wie die Publikation von Gingerella und Jacanin zeigt (Cal Lab, July, August 2000, Seiten 29-36). So wurde in dieser Publikation die Genauigkeit von Leitfähigkeitslösungen mit 10 µS/cm bei 25 °C von verschiedenen Firmen geprüft, wobei die Fehler zwischen 1 und 30 % betrugen.

Das Dokument US-A-4,092,232 offenbart einen H₂S Gassensor mit einem Referenzelektrolyten, der eine Lösung aus Ethylenglykol, Wasser (70:30 Verhältnis) und Kaliumchlorid (0,005 M) enthält, die mit Silberchlorid gesättigt wurde. In der Veröffentlichung von Light T. et al; Analytical Chemistry; 65, (1993), S. 181-182 wird eine Kalibrationslösung mit niedriger Leitfähigkeit beschrieben, die Sucrose und Kaliumchlorid enthält.

Die ungenügende Haltbarkeit von Kalibrationslösungen mit tiefer Leitfähigkeit ist verständlich, da sie stets sehr verdünnte Lösungen mit einem Salzgehalt von weniger als 0.0001M sind. Bereits Kontaminationen durch Kohlendioxid der Luft können einen Messfehler von ca. 1 µS/cm bei 25 °C verursachen. Verunreinigungen des Behälters oder des Kalibrationsgefäßes und der Messzelle selbst sind weitere Fehlerquellen.

Die Lösungen des NIST mit 5 und 15 µS/cm bei 25 °C basieren auf einer wässrigen KCI Lösung, der 30 % Propanol zugesetzt wurde. Bei nur teilweise wässrigen Lösungen kann der Salzgehalt leicht erhöht werden im Vergleich zu entsprechenden rein wässrigen Lösungen. Diese Lösungen der NIST weisen trotzdem eine Haltbarkeit von weniger als einem Jahr auf. Auch ist gemäß den Anforderungen der USP-Vorschriften ihre Genauigkeit unbefriedigend und zu niedrig.

Zusammenfassend weisen die heutigen Kalibrationslösungen im tiefen Leitfähigkeitsbereich folgende Einschränkungen auf:
eine ungenügende Genauigkeit sowie eine kurze Haltbarkeit und ein hohes Kontaminationsrisiko aufgrund einer zu geringen Salzkonzentration.

Der vorliegenden Erfindung liegt deshalb die Aufgabe zugrunde, die Nachteile der bestehenden Kalibrationslösungen mit niedriger Leitfähigkeit zu eliminieren, insbesondere stabile Lösungen mit hoher Genauigkeit, langer Haltbarkeit und niedrigem Kontaminationsrisiko in niedrigem Leitfähigkeitsbereich bereitzustellen. Zur Lösung dieser Aufgabe stellt die vorliegende Erfindung eine Lösung zur Kalibration von konduktometrischen Messzellen bereit, die dadurch gekennzeichnet ist, dass sie mindestens einen Zusatzstoff umfasst, der mindestens zwei Wasserstoffbrückenbindungen bildende Gruppierungen aufweist, wobei die Lösung mindestens ein Salz umfasst und der Gehalt an Salz mindestens 0,0005 M und gleichzeitig bei 25 °C die Leitfähigkeit der Lösung < 70 µS/cm beträgt. Überraschenderweise wurde festgestellt, dass durch die erfindungsgemäß eingesetzten Zusatzstoffe Kalibrationslösungen mit hoher Genauigkeit und Haltbarkeit erhalten werden können.

Die elektrolytische Leitfähigkeit von Lösungen wird durch die Anzahl und Beweglichkeit der einzelnen Ionen festgelegt. Kalibrationslösungen enthalten deshalb üblicherweise Salze, die in Ionen dissoziiert vorliegen, z.B. KCI, Bei den neuartigen Kalibrationslösungen der vorliegenden Erfindung wird die lonenbeweglichkeit der einzelnen Ionen durch die Beigabe von Zusatzstoffen herabgesetzt, die die Bildung von Wasserstoffbrücken verursachen. Dies erlaubt im Gegensatz zu den Kalibrationslösungen des Standes der Technik mit geringer Salzkonzentration überraschenderweise, trotz ausreichend hoher Salzkonzentration, eine niedrige Leitfähigkeit, so dass der Einfluss von Kontamination gering ist. Bevorzugt im Rahmen der vorliegenden Erfindung ist eine Kalibrationslösung mit solchen Zusatzstoffen mit mindestens zwei Wasserstoffbrückenbindungen bildenden Gruppierungen, die die Leitfähigkeit der Lösung durch Erniedrigung der lonenbeweglichkeit um einen Faktor von mindestens 5 erniedrigen im Vergleich zu Lösungen ohne Zusatzstoffe mit mindestens zwei Wasserstoffbrückenbindungen bildenden Gruppen.

In wässrigen Lösungen sind alle Ionen von einer Wasserhülle umgeben. Deshalb werden sie als aquotisierte Ionen bezeichnet. Bei der lonenwanderung wird diese Wasserhülle stets mitgeschleppt. Beispielsweise hat das kleine Lithium-Ion hat eine große Wasserhülle und wandert langsamer als das größere Kalium-Ion mit seiner kleinen Wasserhülle.

Bei den Kalibrationslösungen der vorliegenden Erfindung ist die lonenbeweglichkeit bevorzugt dadurch erniedrigt, indem ein Teil oder alles der Wasserhülle durch andere Moleküle ersetzt wird.

Dies wird erfindungsgemäß bevorzugt mit Zusatzstoffen erreicht, welche starke Wasserstoffbrücken bilden. Besonders bevorzugt werden Zusatzstoffe mit solchen Wasserstoffbrückenbindungen bildende Gruppierungen eingesetzt, die Wasserstoffbrückenbindungen eingehen, deren Bindungsenergie mindestens 1 kJ/mol und maximal 50 kJ/mol beträgt.

Als besonders geeignet hat sich der Einsatz solcher Zusatzstoffe mit mindestens zwei Wasserstoffbrückenbindungen bildenden Gruppierungen erwiesen, deren Gruppierungen ausgewählt sind aus der Gruppe bestehend aus:
Chloro, Fluoro, Hydroxy, C₁ - C₄ Alkoxy, Carboxy, Carbonyl, C₁ - C₄ Alkoxycarbonyl, Amino, C₁ - C₄ Alkylamino, Di-(C₁-C₄-Alkyl)amino, Cyano, Carboxyamid, Carboxy-(C₁-C₄-Alkyl)amino, Carboxy-di(C₁-C₄Alkyl)amino, Sulfo, Sulfido(C₁-C₄-Alkyl), Sulfoxido(C₁-C₄-Alkyl), Sulfono(C₁-C₄-Alkyl), Thio, Nitril, Ester, Nitro, Disulfo, Disulfido, Thioether, Diazoamino, Triazeno, Tetrazano, Azido, Diazo, Diazirin3-carboxy, Pyridazino, Hydrazino, Hydrazo, Aminooxy, Anilino, p-Toluidino, p-Anisidino-, p-Phenetidino, Benzidino, o-Tolidino, N-Methylanilino, Ethylendioxy, Carbonitril, Cyano, Benzosulfonamido, Sulfanilamido, 4-Sulfamoylanilino, Amidino, Carboxamido, Acetamido, Guanidino, Semicarbazido, Semicarbazono, Ureido, Acyl, Diacyl, Oxamoyl, Malonamoyl, Succinamoyl, Phtalamoyl, Carbamoyl, Phenylcarbamoyl, Carbazoyl, Allophanoyl, Hydantoyl, Acetoacetyl, Aminosäuren, Nucleinsäuren und/oder Proteinen.

Insbesondere bevorzugt im Rahmen der vorliegenden Erfindung sind OH-Gruppen als Wasserstoffbrückenbindungen bildende Gruppierungen, da diese leicht erhältlich, kostengünstig, in der Regel nicht toxisch sind und mit Wasser eine starke Wechselwirkung eingehen.

Geeignete Zusatzstoffe im Sinne der vorliegenden Erfindung sind Ethylenglykol, 1,2-Propylenglykol, Diethylenglykol, Glycerin, Triethanolamin, Zucker und/oder Dextran. Auch größere Moleküle wie Polyethylenglykol, Polypropylenglykol, Polyvinylalkohol, di-, trihydroxyendständige Polyether und/oder di-, trihydroxyendständige Polyester sind geeignet, da sie mit Wasser starke Wechselwirkungen eingehen.

Als besonders geeignet hat sich der Einsatz der Zusatzstoffe Triethanolamin, Glycerin, Polyethylenglykol und/oder Ethylenglykol, da diese Zusatzstoffe trotz einer im Vergleich zum Stand der Technik zehnfach höheren Kaliumchloridkonzentration von 0,001 M ausgesprochen niedrige Leitfähigkeiten im Bereich von 1,8 bis 18,9 µS/cm bei 25 °C zeigen. Insbesondere bevorzugt im Rahmen der vorliegenden Erfindung ist der Zusatzstoff Triethanolamin, da mit diesem eine äußerst geringe Leitfähigkeit von nur 1,8 µS/cm bei 25 °C und einer Salzkonzentration von 0,001 M erreicht werden kann. Weiterhin besonders bevorzugt ist Glycerin als Zusatzstoff. Besonders bevorzugt ist eine Kalibrationslösung enthaltend neben einem Salz 60 - 95 Volumen-% Glycerin und 5 - 40 Volumen-% Wasser.

Als niedrige Leitfähigkeit wird ein Bereich von 0,5 bis 146 µS/cm, als mittlere Leitfähigkeit ein Bereich von 147 bis 12.880 µS/cm und als hohe Leitfähigkeit ein Bereich > 12.880 µS/cm definiert. Ein besonderer Vorteil der erfindungsgemäßen Kalibrationslösung im Vergleich zum Stand der Technik liegt darin, dass sie trotz ihres relativ hohen Salzgehaltes von beispielsweise 10⁻³ M eine sehr niedrige Leitfähigkeit realisieren, während im Stand der Technik Kalibrationslösungen beschrieben sind, die entweder zwar niedrige Leitfähigkeiten zeigen, dann jedoch gleichzeitig mit einem sehr verdünnten Salzgehalt von weniger als 0,0001 M einhergehen oder bei einem Salzgehalt von 0,001 M eine Leitfähigkeit von ≥ 146,9 µS/cm zeigen. Erfindungsgemäß weist eine Kalibrationlösung bei 25 °C eine Leitfähigkeit von < 70 µS/cm auf, insbesondere bevorzugt von < 2 µS/cm.

Der Gehalt an Zusatzstoffen beträgt bevorzugt mindestens 10 Volumen-% und maximal 99 Volumen-%, besonders bevorzugt mindestens 50 Volumen-% und maximal 95 Volumen-%, insbesondere bevorzugt mindestens 80 und maximal 90 Volumen-% und am meisten bevorzugt etwa 90 Volumen-%, jeweils bezogen auf die Kalibrationslösung, da bei diesem Gehalt erfindungsgemäß eine besonders niedrige Leitfähigkeit nachgewiesen werden konnte.

Besonders bevorzugt ist im Rahmen der vorliegenden Erfindung eine Kalibrationslösung, die als Lösungsmittel Wasser umfasst, also eine wässrige Kalibrierungslösung. Der Gehalt an Wasser liegt dabei bevorzugt bei einer Konzentration von mindestens 5 Volumen-% und maximal 95 Volumen-%, besonders bevorzugt bei einer Konzentration von mindestens 7 Volumen-% und maximal 50 Volumen-%, insbesondere bevorzugt bei einer Konzentration von mindestens 10 Volumen-% und maximal 20 Volumen-% und am meisten bevorzugt bei einer Konzentration von etwa 10 Volumen-%, jeweils bezogen auf die Kalibrationslösung. Bevorzugt ist in einer Ausführungsform Wasser das einzige vorhandene Lösungsmittel.

In einer weiteren Ausführungsform der Erfindung umfaßt die Kalibrationslösung als Lösungsmittel Wasser und mindestens ein weiteres Lösungsmittel, wobei das Lösungsmittel ausgewählt ist aus der Gruppe bestehend aus Alkohol, Keton, Ester, Amid und/oder Stickstoffverbindungen. Der Gehalt an dem mindestens einen weiteren Lösungsmittel liegt bevorzugt bei mindestens 10 Volumen-% und maximal 65 Volumen-%, besonders bevorzugt bei mindestens 20 Volumen-% und maximal 40 Volumen-% sind insbesondere bevorzugt bei 30 Volumen-%, jeweils bezogen auf das Lösungsmittelgemisch umfassend Wasser und mindestens ein weiteres Lösungsmittel.

Geeignete Alkohole sind erfindungsgemäß Methanol, Ethanol, Propanol, Butanol, Octanol und/oder Cyclohexanol; geeignete Ketone Aceton, Butanon und/oder Cyclohexanon; geeignete Ester Essigsäureester und/oder Glykolester; ein geeignetes Amid ist das Dimethylformamid; geeignete Stickstoffverbindungen sind Pyridin, N-Methylpyrrolidin, Nitrobenzol und/oder Acetonitril. Insbesondere bevorzugt im Rahmen der vorliegenden Erfindung ist die Verwendung von Alkohol als Lösungsmittel, da dieser leicht erhältlich, kostengünstig und in der Regel nicht toxisch ist. Der Vorteil bei der Verwendung von Lösungen, die zusätzlich zu Wasser noch mindestens ein weiteres Lösungsmittel umfassen, liegt darin, dass der Salzgehalt im Vergleich zu rein wässrigen Lösungen weiter erhöht werden kann.

In einer bevorzugten Ausführungsform der vorliegenden Erfindung ist vorgesehen, dass die Kalibrationslösung mindestens ein Salz umfasst. Dieses Salz ist für den elektrischen Stromtransport in der Lösung nach Dissoziation des Salzes in seine Ionen erforderlich. Durch den Salzzusatz sind die Lösungen außerdem weniger anfällig für Verschmutzungen und weisen dadurch eine längere Haltbarkeit auf. So wurde festgestellt, dass Flaschen gefüllt mit einer erfindungsgemäßen Kalibrationslösung eine Stunde lang offen stehengelassen werden konnten, ohne dass sich die Leitfähgikeit der Lösung durch eindringendes CO₂ signifikant veränderte. Somit hat der Salzgehalt auch einen Einfluss auf die Stabilität der Leitfähigkeit einer Kalibrationslösung. Im Rahmen der vorliegenden Erfindung wurde auch festgestellt, dass sich die Langzeitstabilität einer 5 µS/cm Kalibrationslösung, die in einer 250 ml Glasflasche aufbewahrt wurde, bezüglich ihrer Leitfähigkeit in einem Zeitraum über 27 Monate nur um 0,03 µS/cm bei 25 °C, also nur sehr geringfügig, veränderte. Dies stellt einen großen Vorteil gegenüber den Kalibrationslösungen im Stand der Technik dar, die in der Regel eine Haltbarkeit von weniger als einem Jahr aufweisen.

Besonders bevorzugt im Rahmen der vorliegenden Erfindung sind Salze, die als Kationen Alkali- und/oder Erdalkaliionen umfassen wie beispielsweise Lithium-, Natrium-, Kalium- , Rubidium- oder/und Cäsiumionen und/oder Magnesium-, Calcium-, Strontium-, Barium- oder/und Berilliumionen, aber auch NH₄⁺. Bevorzugte Anionen sind Chloride, Carbonate, Bicarbonate, Phosphate etc. Insbesondere bevorzugt ist das Salz Kaliumchlorid.

Weiterhin ist der Gehalt an Salz mindestens 0,0005 M, bevorzugt mindestens 0,0008 M und insbesondere bevorzugt mindestens 0,001 M.

In einer Ausführungsform der vorliegenden Erfindung liegt die Leitfähigkeit der Kalibrationslösung bei den oben genannten Salzkonzentrationen bevorzugt bei 25 °C bei < 20 µS/cm, noch mehr bevorzugt von < 10 µS/cm und am meisten bevorzugt bei < 2 µS/cm.

Besonders bevorzugt ist eine Lösung, die einen Salzgehalt, z.B. KCI, von ≥ 0,0005 M aufweist und deren Leitfähigkeit gleichzeitig < 50 µS/cm beträgt.

Außerdem ist ein Verfahren zur Herstellung einer Lösung zur Kalibration von konduktometrischen Messzellen vorgesehen, welches dadurch gekennzeichnet ist, dass es die folgenden Schritte umfasst:
(a) Bereitstellen einer wässrigen Kalibrationslösung,
(b) Zugabe mindestens eines Zusatzstoffes zur wässrigen Kalibrationslösung von (a), der mindestens zwei Wasserstoffbrückenbindungen bildende Gruppierungen, wie oben beschrieben, aufweist.

Des Weiteren sieht die vorliegende Erfindung die Verwendung einer Lösung gemäß Anspruch 1 zur Kalibration von konduktometrischen Messzellen vor.

Weiterhin sieht die vorliegende Erfindung die Verwendung von mindestens einem Zusatzstoff, welcher mindestens zwei Wasserstoffbrückenbindungen bildende Gruppierungen aufweist, zur Herstellung einer Lösung zur Kalibration von konduktometrischen Messzellen vor.

Die nachfolgenden Beispiele verdeutlichen die angesprochenen Vorteile der erfindungsgemäßen Kalibrationslösung zur Konduktometrie.

### Beispiel 1

Als Basis dient eine wässrige 0.001 M KCl Lösung mit einer Leitfähigkeit von 147 µS/cm bei 25 °C, wie sie in etlichen Normen beschrieben ist.
0.001 M KCl in 10 % Wasser und 90 % Ethylenglykol:

| | |
|---|---|
| Leitfähigkeit: | 18.9 µS/cm bei 25 °C |

0.001 M KCl in 20 % Wasser und 80 % Glycerin:

| | |
|---|---|
| Leitfähigkeit: | 3.4 µS/cm bei 25 °C |

0.001 M KCl in 20 % Wasser und 80 % Polyethylenglykol:

| | |
|---|---|
| Leitfähigkeit: | 16.3 µS/cm bei 25 °C |

0.001 M KCl in 10 % Wasser und 90 % Triethanolamin:

| | |
|---|---|
| Leitfähigkeit: | 1.8 µS/cm bei 25°C |

Die Ausführungsbeispiele zeigen, dass der Zusatz von geeigneten Substanzen die lonenbeweglichkeit signifikant erniedrigt. Dadurch können Kalibrationslösungen mit sehr tiefen Leitfähigkeit realisiert werden, welche trotzdem einen relativ hohen Salzgehalt von z.B. 10⁻³M aufweisen.

### Beispiel 2

Da der Salzgehalt relativ hoch ist, ist der Einfluss von Verschmutzungen entsprechend geringer. 250 ml Flaschen gefüllt mit diesen Kalibrationslösungen können 1 Stunde offen stehen gelassen werden, ohne dass sich die Leitfähigkeit durch eindringendes CO₂ signifikant verändert.

### Beispiel 3

Das folgende Beispiel zeigt die Langzeitstabilität einer 5 µS/cm Kalibrationslösung, welche in einer 250 ml Glasflasche aufbewahrt wurde.
Periodisch bestimmte man die Leitfähigkeit bei 25 °C.

| Datum | Leitfähigkeit |
|---|---|
| September 2000 | 4.97 µS/cm bei 25 °C |
| April 2001 | 4.98 µS/cm bei 25 °C |
| Dezember 2001 | 4.99 µS/cm bei 25 °C |
| Juni 2002 | 4.99 µS/cm bei 25 °C |
| Dezember 2002 | 5.00 µS/cm bei 25 °C |

Einige dieser Werte wurden durch die Physikalisch-Technische Bundesanstalt in Braunschweig bestätigt.

## Patentansprüche

1. Lösung zur Kalibration von konduktometrischen Messzellen,
**dadurch gekennzeichnet,**
**dass** sie mindestens einen Zusatzstoff umfasst, der mindestens zwei Wasserstoffbrückenbindungen bildende Gruppierungen aufweist, ausgewählt aus der Gruppe bestehend aus Ethylenglykol, 1,2-Propylenglykol, Diethylenglykol, Glycerin, Triethanolamin, Polyethylenglykol, Polypropylenglykol, Polyvinylalkohol, di-, trihydroxyendständige Polyether und/oder di-,-trihydroxyendständige Polyester,
wobei die Lösung mindestens ein Salz umfasst und der Gehalt an Salz mindestens 0,0005 M und gleichzeitig bei 25 °C die Leitfähigkeit der Lösung < 70 µS/cm beträgt.

2. Lösung nach Anspruch 1,
**dadurch gekennzeichnet,**
**dass** die Wasserstoffbrückenbindungen bildenden Gruppierungen Wasserstoffbrückenbindungen eingehen, deren Bindungsenergie mindestens 1 kJ/mol und maximal 50 kJ/mol beträgt.

3. Lösung nach einem der Ansprüche 1 bis 2,
**dadurch gekennzeichnet,**
**dass** die Wasserstoffbrückenbindungen bildenden Gruppierungen OH-Gruppen sind.

4. Lösung nach einem der Ansprüche 1 bis 3,
**dadurch gekennzeichnet,**
**dass** der Zusatzstoff Triethanolamin, Glycerin, Polyethylenglykol und/oder Ethylenglykol ist.

5. Lösung nach Anspruch 4,
**dadurch gekennzeichnet,**
**dass** der Zusatzstoff Triethanolamin oder/und Glycerin ist.

6. Lösung nach einem der Ansprüche 1 bis 5,
**dadurch gekennzeichnet,**
**dass** sie bei 25 °C eine Leitfähigkeit von < 150 µS/cm aufweist.

7. Lösung nach einem der Ansprüche 1 bis 6,
**dadurch gekennzeichnet,**
**dass** der Gehalt an Zusatzstoffen mindestens 10 Volumen-% und maximal 99 Volumen-%, bezogen auf die Kalibrationslösung, beträgt.

8. Lösung nach einem der Ansprüche 1 bis 7,
**dadurch gekennzeichnet,**
**dass** sie als Lösungsmittel Wasser umfasst.

9. Lösung nach einem der Ansprüche 1 bis 8,
**dadurch gekennzeichnet,**
**dass** sie als Lösungsmittel Wasser und mindestens ein weiteres Lösungsmittel umfasst.

10. Lösung nach Anspruch 9,
**dadurch gekennzeichnet,**
**dass** das mindestens eine weitere Lösungsmittel ausgewählt ist aus der Gruppe bestehend aus Alkohol, Keton, Ester, Amid und/oder Stickstoffverbindungen.

11. Verwendung einer Lösung gemäß Anspruch 1 zur Kalibration von konduktometrischen Messzellen.

## Claims

1. Solution for calibrating conductometric measuring cells,
**characterized in that**
it comprises at least one additive which has at least two groups that form hydrogen bonds which are selected from the group consisting of ethylene glycol, 1,2-propylene glycol, diethylene glycol, glycerol, triethanolamine, polyethylene glycol, polypropylene glycol, polyvinyl alcohol, dihydroxy-terminated and trihydroxy-terminated polyethers and/or dihydroxy-terminated and trihydroxy-terminated polyesters, wherein the solution contains at least one salt and the content of salt is at least 0.0005 M and at the same time the conductivity of the solution is < 70 uS/cm at 25°C:

2. Solution according to claim 1,
**characterized in that**
the groups that form hydrogen bonds form hydrogen bonds whose bonding energy is at least 1 kJ/mol and at most 50 kJ/mol.

3. Solution according to one of the claims 1 to 2,
**characterized in that**
the groups that form hydrogen bonds are OH groups.

4. Solution according to one of the claims 1 to 3,
**characterized in that**
the additive is triethanolamine, glycerol, polyethylene glycol and/or ethylene glycol.

5. Solution according to claim 4,
**characterized in that**
the additive is triethanolamine or/and glycerol.

6. Solution according to one of the claims 1 to 5,
**characterized in that**
it has a conductivity of < 150 µS/cm at 25°C.

7. Solution according to one of the claims 1 to 6,
**characterized in that**
the content of additives is at least 10 % by volume and at most 99 % by volume based on the calibration solution.

8. Solution according to one of the claims 1 to 7,
**characterized in that**
it comprises water as a solvent.

9. Solution according to one of the claims 1 to 8,
**characterized in that**
it comprises water as a solvent and at least one further solvent.

10. Solution according to claim 9,
**characterized in that**
the at least one further solvent is selected from the group consisting of alcohol, ketone, ester, amide and/or nitrogen compounds.

11. Use of a solution according to claim 1 to calibrate conductometric measuring cells.

## Revendications

1. Solution de calibrage de cellules de conductimétrie, **caractérisée en ce qu'**elle comprend au moins un additif présentant au moins deux groupements formateurs de liaisons hydrogène, choisi dans le groupe formé par l'éthylèneglycol, le 1,2-propylèneglycol, le diéthylèneglycol, la glycérine, la triéthanolamine, le polyéthylèneglycol, le polypropylèneglycol, un poly(alcool vinylique), un polyéther comportant 2 ou 3 groupes hydroxyle terminaux et/ou un polyester comportant 2 ou 3 groupes hydroxyle terminaux, la solution comprenant au moins un sel et la teneur en sel étant au moins égale à 0,0005 M et simultanément, la conductibilité de la solution à 25°C s'élèvant à < 70 µS/cm.

2. Solution selon la revendication 1, **caractérisée en ce que** les groupements formateurs de liaisons hydrogène forment des liaisons hydrogène, dont l'énergie de liaison s'élève à au moins 1 kJ/mole et au maximum à 50 kJ/mole.

3. Solution selon l'une des revendications 1 à 2, **caractérisée en ce que** les groupements formateurs de liaisons hydrogène sont des groupes OH.

4. Solution selon l'une des revendications 1 à 3, **caractérisée en ce que** l'additif est la triéthanolamine, la glycérine, le polyéthylèneglycol et/ou l'éthylèneglycol.

5. Solution selon la revendication 4, **caractérisée en ce que** l'additif est la triéthanolamine et/ou la glycérine.

6. Solution selon l'une des revendications 1 à 5, **caractérisée en ce qu'**à une température de 25 °C, elle présente une conductibilité de < 150 µS/cm.

7. Solution selon l'une des revendications 1 à 6, **caractérisée en ce que** la teneur en additifs s'élève à au moins 10% en volume et au maximum 99% en volume, par rapport à la solution de calibration.

8. Solution selon l'une des revendications 1 à 7, **caractérisée en ce qu'**elle comprend de l'eau comme solvant.

9. Solution selon l'une des revendications 1 à 8, **caractérisée en ce qu'**elle comprend de l'eau et au moins un autre solvant comme solvant.

10. Solution selon la revendication 9, **caractérisée en ce que** l'autre solvant est choisi dans le groupe formé par un alcool, une cétone, un ester, un amide et/ou des composés azotés.

11. Utilisation d'une solution selon la revendication 1, pour la calibration de cellules de conductimétrie.
